# EUROPÄISCHE PATENTSCHRIFT

(11) **EP 3 870 989 B1**
(45) Veröffentlichungstag und Bekanntmachung des Hinweises auf die Patenterteilung: **16.04.2025**
(21) Anmeldenummer: 19786815.1
(22) Anmeldetag: 18.10.2019
(51) Int. Cl.: G01R 33/561, G01R 33/56, A61B 5/055

(54) **MAGNETRESONANZ-FINGERPRINTING-VERFAHREN FÜR AUFNAHMEN MIT KONTRASTMITTEL**
MAGNETIC RESONANCE FINGERPRINTING METHOD FOR ACQUISITIONS WITH CONTRAST AGENT
PROCÉDÉ DE PRISE D'EMPREINTES À RÉSONANCE MAGNÉTIQUE POUR DES ACQUISITIONS AVEC UN AGENT DE CONTRASTE

(30) Priorität: 25.10.2018 EP 18202461
(43) Veröffentlichungstag der Anmeldung: 01.09.2021
(73) Patentinhaber: Bayer Aktiengesellschaft, 51373 Leverkusen (DE)
(72) Erfinder: SCHÜTZ, Gunnar, 13467 Berlin (DE); JOST, Gregor, 10318 Berlin (DE)
(74) Vertreter: BIP Patents
(86) Internationale Anmeldenummer: PCT/EP2019/078387
(87) Internationale Veröffentlichungsnummer: WO 2020/083778

(56) Entgegenhaltungen:
- WO-A1-2018/187760
- ALI DEVRIM KARAOSMANOGLU ET AL: "Magnetic Resonance Imaging of Liver Metastasis", SEMINARS IN ULTRASOUND, CT, AND MR, vol. 37, no. 6, 1 December 2016 (2016-12-01), US, pages 533 - 548, XP055583392, ISSN: 0887-2171, DOI: 10.1053/j.sult.2016.08.005
- EMRE UNAL ET AL: "Multiparametric or practical quantitative liver MRI: towards millisecond, fat fraction, kilopascal and function era", EXPERT REVIEW OF GASTROENTEROLOGY AND HEPATOLOGY200806GB, vol. 11, no. 2, 11 February 2017 (2017-02-11), UK, pages 167 - 182, XP055582630, ISSN: 1747-4124, DOI: 10.1080/17474124.2017.1271710
- JOSHUA D KAGGIE ET AL.: "T1 and T2 Mapping of Delayed Gadolinium Enhancement in Osteoarthritis with MR Fingerprinting", PROCEEDINGS OF THE INTERNATIONAL SOCIETY FOR MAGNETIC RESONANCE IN MEDICINE, ISMRM, 20TH ANNUAL MEETING AND EXHIBITION, MELBOURNE, AUSTRALIA, 5-11 MAY 2012, no. 1435, 1 June 2018 (2018-06-01), XP040700643
- BHAIRAV BIPIN MEHTA ET AL: "Magnetic resonance fingerprinting: a technical review : Magnetic Resonance in Medicine", MAGNETIC RESONANCE IN MEDICINE., vol. 81, no. 1, 14 September 2018 (2018-09-14), US, pages 25 - 46, XP055562942, ISSN: 0740-3194, DOI: 10.1002/mrm.27403

## Beschreibung

Die vorliegende Erfindung befasst sich mit dem technischen Gebiet der Magnetresonanztomografie. Gegenstände der vorliegenden Erfindung sind ein Verfahren, ein System und ein Computerprogrammprodukt zur Ermittlung verschiedener Zustände eines Kontrastmittels in verschiedenen Gewebearten in einem Magnetresonanz-Fingerprinting-Verfahren.

Die Magnetresonanztomographie, abgekürzt MRT oder MR (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, sodass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer T1-Verkürzung führen.

Die Wirkung dieser Kontrastmittel ist indirekt, da das Kontrastmittel selbst kein Signal abgibt, sondern nur die Signalintensität in seiner Umgebung beeinflusst.

Ein Beispiel für ein superparamagnetisches Kontrastmittel sind Eisenoxidnanopartikel (SPIO, engl.: *superparamagnetic iron oxide*).

Beispiele für paramagnetische Kontrastmittel sind Gadolinium-Chelate wie Gadopentetat-Dimeglumin (Handelsname: Magnevist^{®} u.a.), Gadotersäure (Dotarem^{®}, Dotagita^{®}, Cyclolux^{®}), Gadodiamid (Omniscan^{®}), Gadoteridol (ProHance^{®}) und Gadobutrol (Gadovist^{®}).

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Die Zellen von Zysten, Metastasen und der meisten Leberzellkarzinome arbeiten nicht mehr wie normale Leberzellen, nehmen das Kontrastmittel nicht oder kaum auf, werden nicht verstärkt dargestellt und werden damit erkennbar und lokalisierbar. Beispiele für Kontrastmittel auf der Basis der Gadoxetsäure sind in US 6,039,931A beschrieben; sie sind kommerziell zum Beispiel unter den Markennamen Primovist^{®} oder Eovist^{®} erhältlich.

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels. Dabei wird die hepatozelluläre Aufnahme durch ein organisches Anion-Transportsystem vermittelt, welches auch an der Aufnahme von Bilirubin in die Leberzelle beteiligt ist (O. Clément et al., Gadolinium-ethoxybenzyl-DTPA, a new liver-specific magnetic resonance contrast agent. Kinetic and enhancement patterns in normal and cholestatic rats; Invest Radiol (1992); 27: 612-619).

A. D. Karaosmanoglu et al. offenbaren ein MRT-Verfahren zum Nachweis von Lebermetastasen unter Verwendung von Gd-EOB-DTPA als Kontrastmittel (Magnetic Resonance Imaging of Liver Metastasis, Semin Ultrasound CT MR, 2016, 37(6): 533-548).

E. Unal et al. offenbaren ein MRT-Verfahren zur Beurteilung der Leberfunktion unter Verwendung von Gd-EOB-DTPA als Kontrastmittel (Multiparametrie or practical quantitative liver MRI: towards millisecond, fat fraction, kilopascal and function era, Expert Review of Gastroenterology & Hepatology, 2017, 11(2), 167-182).

Mittels Magnetresonanz-Fingerprinting-Verfahren lassen sich detailliertere Aufnahmen von einem Untersuchungsobjekt in kürzerer Zeit erzeugen. Magnetresonanz-Fingerprinting-Verfahren sind beispielsweise beschrieben in Ma et al., Magnetic Resonance Fingerprinting, Nature, 495: 187-192 (2013); Jiang et al., MR Fingerprinting Using Fast Imaging with Steady State Precession (FISP) with Spiral Readout, Magnetic Resonance in Medicine 74: 1621-1631 (2015) oder Cloos et al., Online Radial Multiband Magnetic Resonance Fingerprinting, ISMRM 2016: 608.

B. B. Mehta et al.: geben in einem Übersichtsartikel einen technischen Einblick in Magnetresonanz-Fingerprinting (Magnetic resonance fingerprinting: a technical review, Magnetic Resonance in Medicine, 2019, 81(1): 25-46).

Ein Magnetresonanz-Fingerprinting-Verfahren ist ein quantitatives Messverfahren, bei dem zunächst mehrere Magnetresonanz-Bilder eines Untersuchungsobjekts bei variierenden Aufnahmeparametern erzeugt werden. Die Aufnahmeparameter werden dabei üblicherweise in einer pseudorandomisierten Weise variiert. Für die einzelnen Bildpunkte (Voxel) der Magnetresonanz-Bilder wird dann ein ortsabhängiger Magnetresonanz-Signalverlauf generiert. Der ermittelte Signalverlauf wird anschließend für jedes Voxel mit mehreren in einer Datenbank hinterlegten Signalverläufen verglichen, wobei jedem der Signalverläufe in der Datenbank ein spezifischer Wert mindestens eines Gewebeparameters zugeordnet ist. Die Datenbank-Signalverläufe stellen zu erwartende Signalverläufe dar und werden vorab ermittelt und/oder berechnet. Ein Volumenelement einer Probe, bei dem der Wert des mindestens einen Gewebeparameters dem spezifischen Datenbankwert entspricht, sollte in einer MagnetresonanzMessung den in der Datenbank gespeicherten Signalverlauf zeigen. Wird also ein übereinstimmender Signalverlauf identifiziert, so lässt sich aus der Datenbank der dazugehörige Wert des mindestens einen Gewebeparameters auslesen.

Auf diese Weise kann aus MR-Messdaten die räumliche Verteilung von gewebespezifischen Parametern (wie die Transversalrelaxation T2 oder die Longitudinalrelaxation T1; sogenannte T1-und T2-Karten) in dem abgebildeten Untersuchungsobjekt quantitativ ermittelt werden. Ein Vorteil der Magnetresonanz-Fingerprinting-Methode ist dabei, dass mehrere Gewebeparameter gleichzeitig in einer einzelnen Messung akquiriert werden können.

J.D. Kaggie et al. offenbaren ein Magnetresonanz-Fingerprinting-Verfahren bei Patienten, die an einer Kniearthrose leiden (T1 and T2 Mapping of Delayed Gadolinium Enhancement in Osteoarthritis with MR Fingerprinting, Proceedings of the International Society for Magnetic Resonance Medicine, 20th Annual Meeting and Exhibition, Melbourne, Australia, 5-11, Nr. 1435, 1. Juni 2018).

WO2018/187760A1 offenbart ein dynamisches Mehrfachkontrast-Magnetresonanz-Fingerprinting-Verfahren. Das Verfahren umfasst: a) Einbringen von zwei oder mehr Kontrastmitteln in einen Untersuchungsbereich eines Subjekts, wobei die zwei oder mehr Kontrastmittel unterschiedliche Relaxivitäten aufweisen; b) Messen einer T1-Relaxationszeit und einer T2-Relaxationszeit für Orte innerhalb des Untersuchungsbereichs unter Verwendung von Magnetresonanz-Fingerprinting; c) Bestimmen, unter Verwendung von Gleichungen, die die unterschiedlichen Relaxivitäten, die T1-Relaxationszeit, die T2-Relaxationszeit und die Konzentrationen der zwei oder mehr Kontrastmittel in Beziehung setzen, der Konzentrationen der zwei oder mehr Kontrastmittel für jeden der Orte innerhalb des Untersuchungsbereichs; und d) Erzeugen eines Bildes, das den Untersuchungsbereich darstellt, zumindest teilweise basierend auf den Konzentrationen der zwei oder mehr Kontrastmittel.

C. E. Anderson et al. berichten über die gleichzeitige Messung von Gewebeparametern von zwei gleichzeitig verabreichten Kontrastmitteln (Dual Contrast - Magnetic Resonance Fingerprinting (DCMRF): A Platform for Simultaneous Quantification of Multiple MRI Contrast Agents; Scientific Reports 7; 8431: 1-10; DOI: 10.1038/s41598-017-08762-9). Das Verfahren nutzt die Tatsache, dass sich die verwendeten Kontrastmittel in ihren Relaxivitäten unterscheiden und sich daher differenzieren lassen.

Wird ein Kontrastmittel verabreicht, so verteilt es sich im Körper. Eine gemessene Relaxationsänderung wird durch das Kontrastmittel, das sich in verschiedenen Gewebsbereichen befinden kann, verursacht. Die Signale können dabei von verschiedenen Gewebearten stammen (z.B. Funktionsgewebe (Parenchym), Zwischengewebe (Interstitium) und/oder einer Gewebsflüssigkeit (z.B. Blut, Lymphe, Galle).

Gesucht wird nach einer Lösung, mit der verschiedene Gewebsarten, die in einem Volumenelement vorhanden sind, in einer einzigen Messung mit nur einem Kontrastmittel differenziert werden können.

Diese Aufgabe wird durch die Gegenstände der unabhängigen Patentansprüche gelöst. Bevorzugte Ausführungsformen finden sich in den abhängigen Patentansprüchen sowie in der vorliegenden Beschreibung und den Figuren.

Ein erster Gegenstand der vorliegenden Erfindung ist ein Verfahren umfassend die folgenden Schritte:
- Bereitstellen einer Magnetresonanz-Fingerprint-Datenbank,
   - wobei die Magnetresonanz-Fingerprint-Datenbank Datenbank-Signalverläufe für ein spezifisches Kontrastmittel in mindestens zwei verschiedenen Gewebearten, einer ersten Gewebeart und einer zweiten Gewebeart, umfasst, wobei das Kontrastmittel Gadoxetsäure oder ein Salz der Gadoxetsäure als kontrastverstärkenden Wirkstoff umfasst, wobei es sich bei der ersten Gewebeart um Hepatozyten handelt,
   - wobei das Kontrastmittel in der ersten Gewebeart einen anderen Zustand aufweist, als in der zweiten Gewebeart, wobei es sich bei dem Zustand um die molare T1-Relaxivität und/oder T2-Relaxivität handelt,
- Erfassen eines Magnetresonanz-Signalverlaufs für ein Volumenelement eines Untersuchungsbereichs mittels einer Magnetresonanz-Fingerprinting-Methode unter Verwendung des Kontrastmittels, wobei das Volumenelement die erste Gewebeart und die zweite Gewebeart umfasst, wobei der Untersuchungsbereich die Leber oder ein Teil der Leber eines Untersuchungsobjekts ist,
- Empfangen von Datenbank-Signalverläufen aus der Magnetresonanz-Fingerprint-Datenbank, wobei jedem Datenbank-Signalverlauf als Datenbankwerte eine T1-Relaxationszeit und eine T2-Relaxationszeit in beiden Gewebearten zugeordnet ist,
- Vergleichen des Magnetresonanz-Signalverlaufs mit Datenbank-Signalverläufen,
- Identifizieren eines Datenbank-Signalverlaufs mit einer definierten Übereinstimmung mit dem Magnetresonanz-Signalverlauf,
- Ermitteln zugehörigen Datenbankwerte der T1- und T2-Relaxationszeiten in beiden Gewebearten in dem Volumenelement anhand des identifizierten Datenbank-Signalverlaufs. Errechnung der Konzentration des Kontrastmittels in der ersten Gewebeart und der Konzentration des Kontrastmittels in der zweiten Gewebeart für das Volumenelement aus den ermittelten T1- und T2-Relaxationszeiten,
- Ausgeben der ermittelten T1- und T2-Relaxationszeiten und/oder der errechneten Konzentrationen des Kontrastmittels in der ersten Gewebeart und in der zweiten Gewebeart für das Volumenelement.

Ein weiterer Gegenstand der vorliegenden Erfindung ist ein entsprechendes System gemäß Anspruch 7.

Ein weiterer Gegenstand der vorliegenden Erfindung ist ein entsprechendes Computerprogrammprodukt gemäß Anspruch 8.

Die Erfindung wird nachstehend näher erläutert, ohne zwischen den Erfindungsgegenständen (Verfahren, System, Computerprogrammprodukt) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt) sie erfolgen.

Wenn in der vorliegenden Beschreibung Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar, der Schutzumfang der Erfindung ist durch die Ansprüche definiert.

Die vorliegende Erfindung nutzt ein Magnetresonanz-Fingerprinting-Verfahren, um verschiedene Zustände eines Kontrastmittels in unterschiedlichen Gewebearten in einer Messung gleichzeitig zu erfassen.

Unter einer Gewebeart wird eine Ansammlung differenzierter Zellen einschließlich ihrer extrazellulären Matrix verstanden. Beispiele für Gewebearten sind Epithelgewebe, Binde- und Stützgewebe (Knochengewebe, Knorpelgewebe, Fettgewebe), Muskel-Gewebe, Nervengewebe und Lebergewebe. Vorliegend werden auch Gewebsflüssigkeiten bzw. flüssige Gewebe (z.B. Blut, Lymphe, Galle) zu den Gewebearten gezählt. Ferner können krankhaftes und gesundes Gewebe zwei unterschiedliche Gewebearten darstellen.

Unter einem Kontrastmittel wird ein Stoff oder Stoffgemisch verstanden, dessen Anwesenheit in einer Magnetresonanzmessung zu einem veränderten Signal führt. Vorzugsweise führt das Kontrastmittel zu einer Verkürzung der T1-Zeitspanne und/oder der T2-Zeitspanne.

Das Kontrastmittel liegt in mindestens zwei Gewebearten in unterschiedlichen Zuständen vor. Das bedeutet, dass es eine erste Gewebeart und eine zweite Gewebeart gibt. Kontrastmittel, das einem Untersuchungsobjekt verabreicht wird, verteilt sich in den Gewebearten; verabreichtes Kontrastmittel liegt demnach sowohl in der ersten als auch in der zweiten Gewebeart vor. Wie sich das Kontrastmittel auf die Gewebearten verteilt, lässt sich prinzipiell experimentell ermitteln und beispielsweise durch einen Verteilungskoeffizienten angeben. Ein solcher Verteilungskoeffizient kann beispielsweise der Quotient aus der Konzentration des Kontrastmittels in der ersten Gewebeart und der Konzentration des Kontrastmittels in der zweiten Gewebeart sein. Es ist denkbar, dass der Verteilungskoeffizient selbst konzentrationsabhängig ist, das heißt, dass er sich mit der Konzentration des Kontrastmittels in einer Gewebeart ändern kann

Das Kontrastmittel weist in der ersten Gewebeart einen anderen Zustand auf als in der zweiten Gewebeart. Ein anderer Zustand führt zu einer veränderten Relaxation. Ein Zustand kann durch mindestens einen Wert eines Gewebeparameters charakterisiert werden. In einer Ausführungsform bedeuten die Begriffe "anderer Zustand" oder "unterschiedliche (verschiedene) Zustände", dass das Kontrastmittel in der ersten Gewebeart eine andere molare T1-Relaxivität aufweist als in der zweiten Gewebeart. In einer anderen Ausführungsform bedeuten die Begriffe "anderer Zustand" oder "unterschiedliche (verschiedene) Zustände", dass das Kontrastmittel in der ersten Gewebeart eine andere molare T2-Relaxivität aufweist als in der zweiten Gewebeart. In einer weiteren Ausführungsform bedeuten die Begriffe "anderer Zustand" oder "unterschiedliche (verschiedene) Zustände", dass das Kontrastmittel in der ersten Gewebeart sowohl eine andere molare T1-Relaxivität als auch eine andere molare T2-Relaxivität aufweist als in der zweiten Gewebeart.

Die molare Relaxivität ist ein gebräuchliches Maß für die Wirksamkeit, das heißt für die relaxationsverkürzende Wirkung eines Kontrastmittels. Ihre Einheit kann in (s·Mol/L)⁻¹ angegeben werden (Sekunde x Mol / Liter)⁻¹.

Vorzugsweise ist eine molare Relaxivität in der ersten Gewebeart mindestens um den Faktor 1,5 größer als in der zweiten Gewebeart, besonders bevorzugt ist sie mindestens um den Faktor 2 größer, noch mehr bevorzugt ist sie mindestens um den Faktor 2,5 größer. In einer besonders bevorzugten Ausführungsform unterscheiden sich die molaren Relaxivitäten in den zwei verschiedenen Gewebearten um einen Faktor von mindestens 1,5 (vorzugsweise mindestens 2, noch mehr bevorzugt mindestens 2,5) bei einer Magnetfeldstärke des Grundmagnetfeldes von mindestens 1,5 Tesla, vorzugsweise bei mindestens 2 Tesla, noch mehr bevorzugt bei mindestens 2,5 Tesla, noch mehr bevorzugt bei mindestens 3 Tesla, am meisten bevorzugt bei einer Magnetfeldstärke von 0,4 Tesla bis mindestens 3 Tesla.

Bei dem Kontrastmittel handelt es sich um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium). Gd-EOB-DTPA weist in Leberzellen (Hepatozyten) eine signifikant höhere molare Relaxivität auf als beispielsweise in Plasma und in Plasma eine signifikant höhere molare Relaxivität als in Gallenflüssigkeit.

Tabelle 1 zeigt molare Relaxivitäten (in (s·mMol/L)⁻¹) von Gd-EOB-DTPA in verschiedenen Gewebearten bei unterschiedlichen Magnetfeldstärken des Grundmagnetfeldes.

**Tab. 1**

| | 0,47 T | 1,41 T | 1,5 T | 3 T |
|---|---|---|---|---|
| Wasser | 5,3⁽¹⁾ | | 4,7⁽¹⁾ | 4,3⁽¹⁾ |
| Galle | | 5,6 ± 0,3 | 5,6 ± 0,4 | 5,6 ± 0,3 |
| Urin | | 5,5 ± 0,1 | 5,5 ± 0,2 | 5,6 ± 0,1 |
| Plasma | 8,7⁽¹⁾ | | 6,9⁽¹⁾ | 6,2⁽¹⁾ |
| Blut | 11,0⁽²⁾ | 8,1 ± 0,2 | 7,3⁽¹⁾/8,3 ± 0,3 | 7,2 ± 0,1 |
| Lebergewebe | 16,6⁽²⁾ | 14,0 ± 2,8 | 16,4 ± 3,1 | 13,4 ± 4,0 |

| | | | | |
|---|---|---|---|---|
| (1): Rohrer et al. Radiol 2005; 40(11): 715-724 (2): Schuhmann-Giampieri et al. Radiol. 1992; 183: 59-64 | | | | |

Überraschend ist, dass die hohe molare Relaxivität in Lebergewebe auch bei hohen (klinisch relevanten) Magnetfeldstärken erhalten bleibt.

Die Erfindung ermöglicht es, Kontrastmittel, das sich in der ersten Gewebeart befindet, von Kontrastmittel, das sich in der zweiten Gewebeart befindet, zu unterscheiden, selbst wenn sich beide Gewebearten in demselben Volumenelement befinden, das in Magnetresonanz-Bilddaten als ein Voxel abgebildet wird.

Das Untersuchungsobjekt ist üblicherweise ein Lebewesen, vorzugsweise ein Säugetier, ganz besonders bevorzugt ein Mensch. Der Untersuchungsbereich ist ein Teil des Untersuchungsobjekts, zum Beispiel ein Organ wie die Leber oder ein Teil eines Organs.

Der Untersuchungsbereich, auch Aufnahmevolumen (engl.: *field of view,* FOV) genannt, stellt insbesondere ein Volumen dar, welches in den aufgenommenen Magnetresonanz-Bilddaten abgebildet wird. Der Untersuchungsbereich wird typischerweise durch einen Nutzer, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

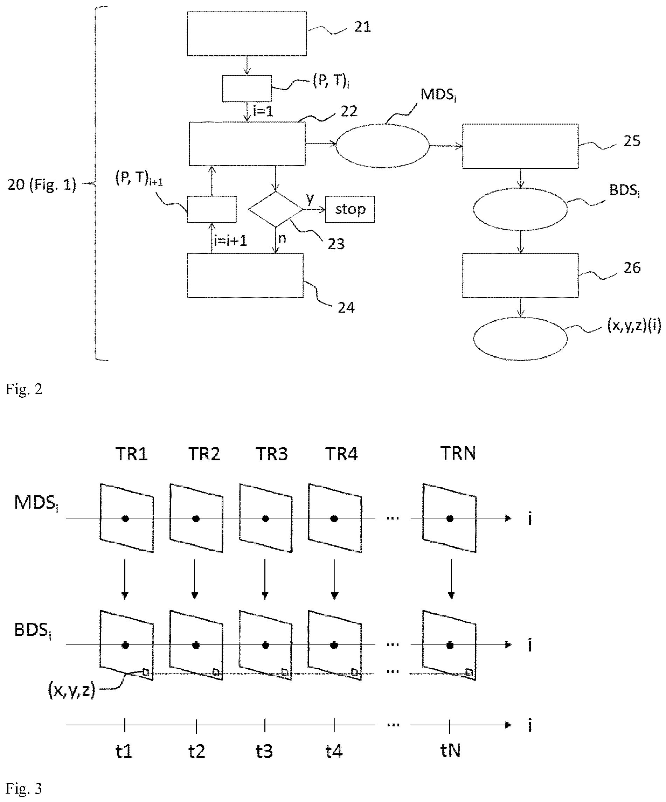

Dem Untersuchungsobjekt wird ein Kontrastmittel verabreicht, das sich in dem Untersuchungsbereich verteilt. Der Untersuchungsbereich wird in ein Grundmagnetfeld eingebracht. Der Untersuchungsbereich wird einem Magnetresonanz-Fingerprinting-Verfahren unterzogen und es wird für mindestens ein Volumenelement des Untersuchungsbereichs ein Magnetresonanz-Signalverlauf ermittelt (Details hierzu siehe Fig. 2 und die dazu gehörige Beschreibung).

Die Magnetresonanz-Fingerprinting-Methode sieht typischerweise ein Erfassen eines Magnetresonanz-Signalverlaufs für eine Vielzahl von Voxeln mittels eines pseudorandomisierten bzw. inkohärenten Aufnahmeschemas vor. Es ist auch denkbar, dass der Magnetresonanz-Signalverlauf aus einem Bereich erfasst wird, welcher gröber aufgelöst als ein Voxel ist. Dann kann der Magnetresonanz-Signalverlauf beispielsweise gemittelt über mehrere Voxel erfasst werden.

Die Magnetresonanz-Fingerprinting-Methode umfasst insbesondere, dass für die Aufnahme der verschiedenen Magnetresonanz-Signale verschiedene Aufnahmeparameter gesetzt werden. Die Aufnahmeparameter können dabei in einer pseudorandomisierten bzw. inkohärenten Weise variiert werden. Mögliche Aufnahmeparameter, welche bei der Akquisition des Magnetresonanz-Signalverlaufs verändert werden, sind beispielsweise eine Echozeit, eine Ausbildung und/oder Anzahl von Hochfrequenz-Pulsen, eine Ausbildung und/oder Anzahl von Gradientenpulsen, eine Diffusionskodierung usw. Auf diese Weise kann mittels der Magnetresonanz-Fingerprinting-Methode ein für das Voxel charakteristischer Magnetresonanz-Signalverlauf, ein sogenannter Fingerabdruck (Fingerprint) des Voxels, erfasst werden.

In einem weiteren Schritt des erfindungsgemäßen Verfahrens werden die Magnetresonanz-Signalverläufe mit Signalverläufen, die in einer Magnetresonanz-Fingerprint-Datenbank gespeichert sind, verglichen (Datenbank-Signalverläufe).

Die Datenbank-Signalverläufe können beispielsweise in einer Kalibrierungsmessung ermittelt werden und/oder simuliert werden. Verfahren zur Erzeugung von Datenbank-Signalverläufen sind im Stand der Technik beschrieben (siehe zum Beispiel J. Xie et al.: Fast dictionary generation and searching for magnetic resonance fingerprinting, Conf Proc IEEE Eng Med Biol Soc. 2017 Jul;2017:3256-3259. doi: 10.1109/EMBC.2017.8037551).

Die Magnetresonanz-Fingerprinting-Methode sieht typischerweise vor, dass ein Datenbank-Signalverlauf dem erfassten Magnetresonanz-Signalverlauf anhand des Ergebnisses des Signalvergleichs zugeordnet wird. Der Signalvergleich kann dabei eine Bestimmung einer Ähnlichkeit des erfassten Magnetresonanz-Signalverlaufs mit den mehreren Datenbank-Signalverläufen umfassen, wobei derjenige Datenbank-Signalverlauf dem Magnetresonanz-Signalverlauf zugeordnet wird, welcher die größte Ähnlichkeit mit dem Magnetresonanz-Signalverlauf aufweist.

Es ist auch denkbar, dass ein Ähnlichkeitsschwellenwert festgelegt wird, der erreicht werden muss, um eine Zuordnung vorzunehmen.

Verfahren zum Vergleich und zur Identifizierung von Ähnlichkeiten sind im Stand der Technik beschrieben (siehe z.B. S.F. Cauley et al., Fast group matching, for MR, fingerprinting reconstruction, Magnetic Resonance in Medicine 74:523-528 (2015)).

Den verschiedenen Datenbank-Signalverläufen sind jeweils spezifische Datenbankwerte von Gewebeparametern zugeordnet.

Die zu dem zugeordneten Datenbank-Signalverlauf gehörenden Datenbankwerte können dann beispielsweise als Messwerte von Gewebeparametern des betrachteten Volumenelements gesetzt werden. Als Ergebnis des Signalvergleichs können zum Beispiel die mittels der Magnetresonanz-Fingerprinting-Methode gewonnenen Messwerte der Gewebeparameter für das entsprechende Voxel ausgegeben bzw. abgespeichert werden.

Eine Auswahl möglicher Gewebeparameter, welche mittels der Magnetresonanz-Fingerprinting Methode quantifiziert werden können, ist: eine T1-Relaxationszeit, eine T2-Relaxationszeit, ein Diffusionswert (beispielsweise ein scheinbarer Diffusionskoeffizient, *apparent diffusion coefficient,* ADC), ein Magnetisierungsmoment, eine Protonendichte, eine Resonanzfrequenz, eine Konzentration eines Stoffs, usw. Selbstverständlich sind auch weitere, dem Fachmann als sinnvoll erscheinende Gewebeparameter denkbar.

Erfindungsgemäß stehen die Datenbankwerte bzw. Messwerte der Gewebeparameter im Zusammenhang mit den verschiedenen Zuständen des verwendeten Kontrastmittels in den mindestens zwei verschiedenen Gewebearter

Erfindungsgemäß umfassen die Datenbankwerte T1- und T2-Relaxationszeiten beider Gewebearten in dem Volumenelement.

Wenn in einem betrachteten Volumenelement erfindungsgemäß zwei verschiedene Gewebearten vorhanden sind und nach Verabreichung eines Kontrastmittels ein Magnetresonanz-Signalverlauf für das Volumenelement erfasst wird, werden erfindungsgemäß durch die Identifizierung des zugehörigen Datenbank-Signalverlaufs die T1-Relaxationszeit und die T2-Relaxationszeit gleichzeitig für beide Gewebearten als zugehörige Datenbankwerte ermittelt und daraus die Konzentration des Kontrastmittels in der ersten Gewebeart und die Konzentration des Kontrastmittels in der zweiten Gewebeart für das entsprechende Volumenelement errechnet.

Es können die ermittelten Datenbankwerte und/oder aus den Datenbankwerten abgeleitete Werte abgespeichert und/oder ausgegeben werden. Ein abgeleiteter Wert ist üblicherweise ein Wert, der sich durch eine Berechnung aus dem Datenbankwert ergibt. Ein Beispiel für einen abgeleiteten Wert ist die Konzentration des Kontrastmittels in einer Gewebeart

Ein Gegenstand der vorliegenden Erfindung ist ein System, mit dem das erfindungsgemäße Verfahren durchgeführt werden kann.

Das System umfasst eine Empfangseinheit, eine Steuereinheit, eine Signalvergleichseinheit und eine Ausgabeeinheit. Es ist denkbar, dass die genannten Einheiten Bestandteile eines einzigen Computersystems sind; es ist aber auch denkbar, dass die genannten Einheiten Bestandteile von mehreren separaten Computersystemen sind, die über ein Netzwerk miteinander verbunden sind, um Daten und/oder Steuersignale von einer Einheit zu einer anderen Einheit zu übermitteln.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen.

Beispiele hierfür sind Monitor (Bildschirm), Drucker, Scanner, Maus, Tastatur, Laufwerke, Kamera,

Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Die Eingaben in das Computersystem erfolgen über Eingabemittel wie beispielsweise eine Tastatur, eine Maus, ein Mikrofon und/oder dergleichen. Als Eingabe soll auch die Auswahl eines Eintrags aus einem virtuellen Menü oder einer virtuellen Liste oder das Anklicken eines Auswahlkästchens und dergleichen verstanden werden.

Das erfindungsgemäße System ist konfiguriert, mindestens einen Magnetresonanz-Signalverlauf und mehrere Datenbank-Signalverläufe zu empfangen, den mindestens einen Magnetresonanz-Signalverlauf mit den Datenbank-Signalverläufen zu vergleichen, als Ergebnis des Vergleichs einen Datenbank-Signalverlauf mit einer definierten Übereinstimmung mit dem Magnetresonanz-Signalverlauf zu identifizieren, Datenbankwerte in Form der T1- und T2-Relaxationszeiten beider Gewebearten, die dem identifizierten Datenbank-Signalverlauf zugeordnet sind, zu ermitteln, die Konzentration des Kontrastmittels in der ersten Gewebeart und die Konzentration des Kontrastmittels in der zweiten Gewebeart für das Volumenelement aus den ermittelten T1- und T2-Relaxationszeiten zu errechnen, und die ermittelten Datenbankwerte und/oder die errechneten Konzentrationen abzuspeichern und/oder auszugeben

Die Steuereinheit dient der Steuerung der Empfangseinheit, der Signalvergleichseinheit und der Ausgabeeinheit sowie der Koordinierung der Daten- und Signalflüsse zwischen verschiedenen Einheiten. Es ist denkbar, dass mehrere Steuereinheiten vorhanden sind.

Die Empfangseinheit dient zum Empfang des mindestens einen Magnetresonanz-Signalverlaufs. Der mindestens eine Magnetresonanz-Signalverlauf kann beispielsweise von einer Magnetresonanzanlage übermittelt werden oder aus einem Datenspeicher ausgelesen werden. Die Magnetresonanzanlage kann ein Bestandteil des erfindungsgemäßen Systems sein. Denkbar ist aber auch, dass das erfindungsgemäße System ein Bestandteil einer Magnetresonanzanlage ist.

Die Empfangseinheit dient ferner zum Empfang von mehreren Datenbank-Signalverläufen aus einer Magnetresonanz-Fingerprinting-Datenbank. Die Magnetresonanz-Fingerprinting-Datenbank kann ein Bestandteil des erfindungsgemäßen Systems sein. In der Magnetresonanz-Fingerprinting-Datenbank ist eine Vielzahl an Datenbank-Signalverläufen gespeichert. Jedem Datenbank-Signalverlauf sind spezifische Werte (Datenbankwerte) von Gewebeparametern zugeordnet, wobei es sich erfindungsgemäß um T1- und T2-Relaxationszeiten der beiden Gewebearten in dem Volumenelement handelt. Die Datenbank-Signalverläufe geben den zu erwartenden Signalverlauf einer Probe an, die entsprechende Werte der Gewebeparameter aufweist. Ein Teil der Datenbank-Signalverläufe gibt die zu erwartenden Signalverläufe einer Probe an, bei der in einem Volumenelement mindestens zwei verschiedene Gewebearten vorhanden sind und ein verabreichtes Kontrastmittel in den mindestens zwei Gewebearten verschiedene Zustände aufweist. Der eine Zustand ist durch einen spezifischen Wert (Datenbankwert) eines ersten Gewebeparameters charakterisiert, der andere Zustand durch einen spezifischen Wert (Datenbankwert) eines zweiten Gewebeparameters.

Von der Empfangseinheit werden der mindestens eine Magnetresonanz-Signalverlauf und die mehreren Datenbank-Signalverläufe an die Signalvergleichseinheit übermittelt.

Die Signalvergleichseinheit ist konfiguriert, den mindestens einen Magnetresonanz-Signalverlauf mit verschiedenen Datenbank-Signalverläufen zu vergleichen, um einen Datenbank-Signalverlauf zu identifizieren, der eine definierte Übereinstimmung mit dem Magnetresonanz-Signalverlauf aufweist.

Ist der Datenbank-Signalverlauf identifiziert, werden die dem Datenbank-Signalverlauf zugeordneten Datenbankwerte ermittelt, wobei es sich erfindungsgemäß um die zugehörigen T1- und T2-Relaxationszeiten der beiden Gewebearten in dem Volumenelement handelt.

Die Ausgabeeinheit ist konfiguriert, die ermittelten Datenbankwerte abzuspeichern und/oder auszugeben. Eine Speicherung erfolgt üblicherweise in einem Datenspeicher, der Bestandteil des erfindungsgemäßen Systems ist oder mit diesem über einem Netzwerk verbunden ist. Eine Ausgabe erfolgt üblicherweise auf einem Bildschirm. Es ist denkbar, dass die Ausgabe in Form eines zwei- oder dreidimensionalen Bildes erfolgt. Es ist denkbar, dass das Bild den betrachteten Untersuchungsbereich darstellt und für einzelne Volumenelemente eine Farbe und/oder ein Grauton auf Basis eines oder mehrerer für das Volumenelement ermittelter Datenbankwerte ausgewählt ist.

Es ist denkbar, dass auf Basis der ermittelten Datenbankwerte abgeleitete Werte berechnet werden. Erfindungsgemäß werden durch die Steuereinheit die Konzentrationen des Kontrastmittels in beiden Gewebearten in dem Volumenelement aus den ermittelten T1- und T2-Relaxationszeiten errechnet.

Die Erfindung wird nachstehend anhand von Figuren näher erläutert, ohne die Erfindung auf die in den Figuren gezeigten Merkmale oder Merkmalskombinationen beschränken zu wollen.

Es zeigen:

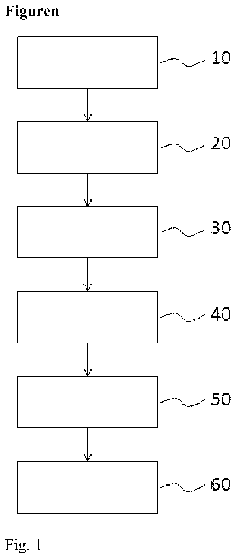

Figur 1 zeigt schematisch einen Ablauf des erfindungsgemäßen Verfahrens in Form einer Abfolge von nacheinander ablaufenden Schritten. Die Abfolge umfasst die Schritte
- Bereitstellen einer Magnetresonanz-Fingerprint-Datenbank (10)
- Erfassen eines Magnetresonanz-Signalverlaufs für ein Volumenelement eines Untersuchungsbereichs mittels einer Magnetresonanz-Fingerprinting-Methode unter Verwendung eines Kontrastmittels (20)
- Vergleichen des Magnetresonanz-Signalverlaufs mit Datenbank-Signalverläufen (30)
- Identifizieren eines Datenbank-Signalverlaufs mit einer definierten Übereinstimmung mit dem Magnetresonanz-Signalverlauf (40)
- Ermitteln der Zustände des Kontrastmittels in dem Volumenelement (50)
- Ausgeben von Informationen zu den Zuständen des Kontrastmittels in dem Volumenelement (60).

Figur 2 zeigt schematisch eine bevorzugte Ausführungsform für das Erfassen eines Magnetresonanz-Signalverlaufs mittels einer Magnetresonanz-Fingerprinting-Methode (Schritt 20 in Figur 1).

Es wird eine Pulssequenz z.B. entsprechend einem gewünschten Kontrast oder anderer gewünschter Eigenschaften der mit der Pulssequenz auslesbaren Messdaten auf übliche Weise gewählt (21).

Die Pulssequenz wird mit einem ersten Satz (i=1) an Aufnahmeparametern Pᵢ durchgeführt, wobei Messdaten entlang einer ersten k-Raum-Trajektorie Tᵢ abgetastet werden sollen (P, T)ᵢ. Eine k-Raum-Trajektorie entlang derer Messdaten in einer Wiederholung gemessen werden, kann den k-Raum kartesisch, spiralförmig, radial oder auch in einer Kombination der vorgenannten Abtastarten oder entlang einer frei erdachten Trajektorie abtasten.

Gemäß der Pulssequenz werden HF-Pulse in den Untersuchungsbereich eingestrahlt, Gradienten geschaltet und die durch die eingestrahlten HF-Pulse und die geschalteten Gradienten erzeugten Echosignale ausgelesen (22). Dabei werden nach einer Anregung mit einem HF-Anregungspuls Messdaten entlang der k-Raum-Trajektorie Tᵢ aufgenommen und in einem Messdatensatz MDSᵢ gespeichert.

Aus jedem Messdatensatz MDSᵢ wird ein Bilddatensatz BDSᵢ rekonstruiert (25), wobei auch nur ein Teil der in dem Messdatensatz MDSᵢ enthaltenen Messdaten für die Rekonstruktion verwendet werden kann. Somit erhält man einen Bilddatensatz BDSᵢ pro Wiederholung i, d.h. insgesamt N Bilddatensätze BDSᵢ. In einer Abfrage (23) wird abgefragt, ob bereits alle N gewünschten Wiederholungen durchgeführt und die entsprechenden N Messdatensätze MSDᵢ gespeichert wurden. Ist dies nicht der Fall ("n"), werden eine k-Raum-Trajektorie für die nächste Wiederholung gewählt und die Parameter der Pulssequenz entsprechend angepasst und ggf. zusätzlich variiert (24). In der Regel wird sich eine gewählte weitere k-Raum-Trajektorie Tᵢ₊₁ von einer vorhergehenden k-Raum-Trajektorie Tᵢ unterscheiden.

Mit dem so erhaltenen nächsten Parametern Pᵢ₊₁ und der gewählten weiteren k-Raum-Trajektorie Tᵢ₊₁ ((P, T)ᵢ₊₁) wird die Pulssequenz wiederholt und somit eine erneute Messung (22) durchgeführt, derart, dass in aufeinanderfolgenden Wiederholungen Messdaten entlang den gewählten k-Raum-Trajektorien Tᵢ, Tᵢ₊₁ gemessen werden. Zur Wahl von Trajektorien sei auf die umfangreiche Literatur zu Magnetresonanz-Fingerprinting-Verfahren verwiesen; als Beispiel sei die Offenlegungsschrift DE102016217675A1 genannt, deren Inhalt durch Bezugnahme vollständig in diese Beschreibung aufgenommen wird.

Wurden bereits alle N gewünschten Wiederholungen durchgeführt und die entsprechenden N Messdatensätze MSDᵢ gespeichert ("y"), wird keine weitere Messung durchgeführt ("stop"), und es wird für mindestens ein Voxel (x,y,z) in den rekonstruierten Bilddatensätzen BDSᵢ eine Voxel-Zeit-Serie (x,y,z)(i) gebildet, die eine Signalintensität des Voxels (x,y,z) im Verlauf der Aufnahmezeiten (und damit im Verlauf der nacheinander durchgeführten Wiederholungen (i) der Messdatensätze MDSᵢ) wiedergibt. Üblicherweise wird eine solche Voxel-Zeit-Serie (x,y,z)(i) für alle Voxel (x,y,z), die in dem interessierenden Untersuchungsbereich liegen, durchgeführt. Die gebildeten Voxel-Zeit-Serien (x,y,z)(i) werden gespeichert.

Figur 3 zeigt schematisch den Zusammenhang von Messdatensätzen MDSᵢ und Bilddatensätzen BDSᵢ im zeitlichen Verlauf, d.h. im Verlauf von i.

In der obersten Zeile sind die Messdatensätze MDSi, wie sie nacheinander in den Wiederholungen TRi aufgenommen wurden, dargestellt, wobei beispielhaft die Wiederholungen i = 1, i = 2, i = 3, i = 4 und i = N explizit dargestellt sind. In der zweiten Zeile sind die aus den Messdatensätzen MDSi rekonstruierten Bilddatensätze BDSi in gleicher Weise dargestellt, wobei beispielhaft ein Voxel (x,y,z) in den Bilddatensätzen BDSi markiert ist. Beispielsweise zu diesem Voxel (x,y,z) kann die jeweilige Intensität des Voxels (x,y,z) zu den, den Wiederholungen Ti entsprechenden Zeiten Ti gegen die Zeit als Voxel-Zeit-Serie aufgetragen werden. Eine Voxel-Zeit-Serie (x,y,z)(i) ist ein Magnetresonanz-Signalverlauf für ein Volumenelement des Untersuchungsbereichs.

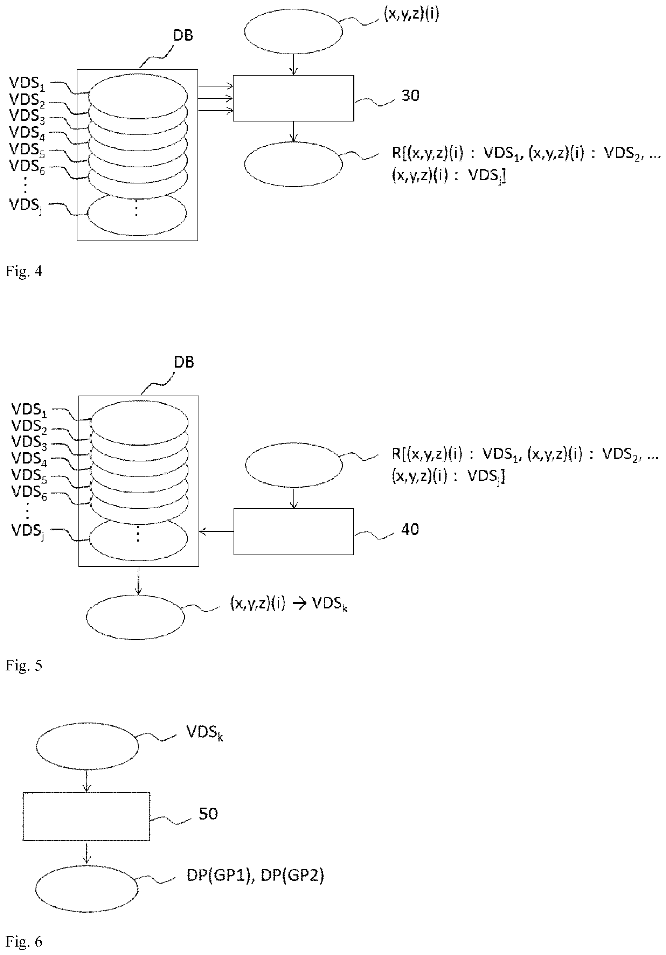

Figur 4 zeigt schematisch den Vergleich von Magnetresonanz-Signalverläufen mit Datenbank-Signalverläufen (Schritt 30 in Figur 1).

Jede gespeicherte Voxel-Zeit-Serie (x,y,z)(i) aus Figur 2 wird mit mehreren der Datenbank-Signalverläufe VDS₁ bis VDSⱼ, die in einer Magnetresonanz-Fingerprinting-Datenbank (DB) gespeichert sind, verglichen (30). Das Ergebnis eines jeden Vergleichs kann ein Ähnlichkeitswert R sein, der angibt, wie ähnlich eine Voxel-Zeit-Serie (x,y,z)(i) einem Datenbank-Signalverlauf ist.

Figur 5 zeigt schematisch die Identifizierung eines Datenbank-Signalverlaufs mit einer definierten Übereinstimmung mit dem Magnetresonanz-Signalverlauf (Schritt 40 in Figur 1).

Üblicherweise wird jeder Voxel-Zeit-Serie (x,y,z)(i) ein Datenbank-Signalverlauf VDSₖ zugeordnet, üblicherweise derjenige Datenbank-Signaverlauf, der die größte Übereinstimmung mit der Voxel-Zeit-Serie (x,y,z)(i) aufweist (maximaler R-Wert).

Figur 6 zeigt schematisch die Ermittlung der Zustände des Kontrastmittels für das Volumenelement, das in der jeweiligen Voxel-Zeit-Serie (x,y,z)(i) abgebildet ist (Schritt 50 in Figur 1). Die Zustände des Kontrastmittels werden durch spezifische Werte von zwei Gewebeparametern GP1 und GP2 charakterisiert. Diese Werte (Datenbankwerte) ergeben sich aus dem in Schritt 50 identifizierten Datenbank-Signaverlauf VDSₖ. Sie sind üblicherweise in der Magnetresonanz-Fingerprinting-Datenbank zum Datenbank-Signaverlauf VDSₖ gespeichert und können nach Identifizierung von VDSₖ ausgelesen werden.

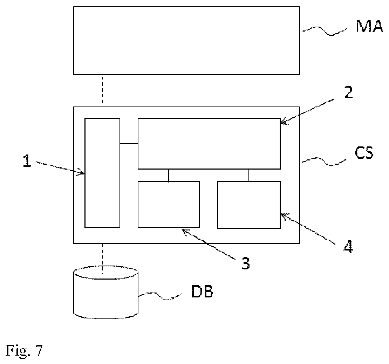

Figur 7 zeigt schematisch eine Ausführungsform des erfindungsgemäßen Systems. Das System umfasst eine Empfangseinheit (1), eine Steuereinheit (2), eine Signalvergleichseinheit (3) und eine Ausgabeeinheit (4). Die genannten Einheiten sind Bestandteile eines Computersystems (CS). Die Empfangseinheit (1) ist konfiguriert, Magnetresonanz-Signalverläufe von einer Magnetresonanzanlage (MA) und Datenbank-Signalverläufe von einer Magnetresonanz-Fingerprinting-Datenbank (DB) zu empfangen. Der Empfang kann über ein Netzwerk (dargestellt durch gestrichelte Linien) erfolgen.

## Patentansprüche

1. Verfahren umfassend die folgenden Schritte:
• Bereitstellen einer Magnetresonanz-Fingerprint-Datenbank (DB),
- wobei die Magnetresonanz-Fingerprint-Datenbank (DB) Datenbank-Signalverläufe (VDS₁, ..., VDSⱼ) für ein spezifisches Kontrastmittel in mindestens zwei verschiedenen Gewebearten, einer ersten Gewebeart und einer zweiten Gewebeart, umfasst, wobei das Kontrastmittel Gadoxetsäure oder ein Salz der Gadoxetsäure als kontrastverstärkenden Wirkstoff umfasst, wobei es sich bei der ersten Gewebeart um Hepatozyten handelt,
- wobei das Kontrastmittel in der ersten Gewebeart einen anderen Zustand aufweist, als in der zweiten Gewebeart, wobei es sich bei dem Zustand um die molare T1-Relaxivität und/oder T2-Relaxivität handelt,
• Erfassen eines Magnetresonanz-Signalverlaufs ((x,y,z)(i)) für ein Volumenelement eines Untersuchungsbereichs mittels einer Magnetresonanz-Fingerprinting-Methode unter Verwendung des Kontrastmittels, wobei das Volumenelement die erste Gewebeart und die zweite Gewebeart umfasst, wobei der Untersuchungsbereich die Leber oder ein Teil der Leber eines Untersuchungsobjekts ist,
• Empfangen von Datenbank-Signalverläufen (VDS₁, ..., VDSᵢ) aus der Magnetresonanz-Fingerprint-Datenbank (DB), wobei jedem Datenbank-Signalverlauf (VDS₁, ..., VDSⱼ) als Datenbankwerte eine T1-Relaxationszeit und eine T2-Relaxationszeit in beiden Gewebearten zugeordnet ist,
• Vergleichen des Magnetresonanz-Signalverlaufs ((x,y,z)(1)) mit Datenbank-Signalverläufen (VDS₁, ..., VDSᵢ),
• Identifizieren eines Datenbank-Signalverlaufs (VDSₖ) mit einer definierten Übereinstimmung mit dem Magnetresonanz-Signalverlauf ((x,y,z)(i)),
• Ermitteln der zugehörigen Datenbankwerte der T1- und T2-Relaxationszeiten in beiden Gewebearten in dem Volumenelement anhand des identifizierten Datenbank-Signalverlaufs (VDSₖ),
• Errechnung der Konzentration des Kontrastmittels in der ersten Gewebeart und der Konzentration des Kontrastmittels in der zweiten Gewebeart für das Volumenelement aus den ermittelten T1- und T2-Relaxationszeiten,
• Ausgeben der ermittelten T1- und T2-Relaxationszeiten und/oder der errechneten Konzentrationen des Kontrastmittels in der ersten Gewebeart und in der zweiten Gewebeart für das Volumenelement

2. Verfahren gemäß Anspruch 1, wobei eine molare Relaxivität in der ersten Gewebeart mindestens um den Faktor 1,5 größer, vorzugsweise mindestens um den Faktor 2 größer, noch mehr bevorzugt mindestens um den Faktor 2,5 größer ist als in der zweiten Gewebeart.

3. Verfahren gemäß Anspruch 2, wobei eine molare Relaxivität bei einer Magnetfeldstärke des Grundmagnetfeldes von mindestens 1,5 Tesla, vorzugsweise mindestens 2 Tesla, noch mehr bevorzugt mindestens 2,5 Tesla, noch mehr bevorzugt mindestens 3 Tesla, am meisten bevorzugt bei einer Magnetfeldstärke von 0,4 Tesla bis mindestens 3 Tesla in der ersten Gewebeart mindestens um den Faktor 1,5 größer, vorzugsweise mindestens um den Faktor 2 größer, noch mehr bevorzugt um mindestens den Faktor 2,5 größer ist als in der zweiten Gewebeart.

4. Verfahren gemäß einem der Ansprüche 1 bis 3, wobei das Kontrastmittel in der ersten Gewebeart eine höhere molare T1-Relaxivität aufweist als in der zweiten Gewebeart.

5. Verfahren gemäß einem der Ansprüche 1 bis 4, wobei das Kontrastmittel Gd-EOB-DTPA Dinatrium umfasst.

6. Verfahren gemäß einem der Ansprüche 1 bis 5, wobei es sich bei der ersten Gewebeart um gesundes Lebergewebe und bei der zweiten Gewebeart um krankhaftes Lebergewebe handelt.

7. System umfassend
• eine Empfangseinheit (1),
• eine Steuereinheit (2),
• eine Signalvergleichseinheit (3) und
• eine Ausgabeeinheit (4),
- wobei die Steuereinheit (2) konfiguriert ist, die Empfangseinheit (1) zu veranlassen, für mindestens ein Volumenelement eines Untersuchungsbereichs einen Magnetresonanz-Signalverlauf ((x,y,z)(i)) zu empfangen, wobei der Magnetresonanz-Signalverlauf ((x,y,z)(i)) in einem Magnetresonanz-Fingerprinting-Verfahren unter Verwendung eines Kontrastmittels generiert worden ist, wobei das Kontrastmittel Gadoxetsäure oder ein Salz der Gadoxetsäure als kontrastverstärkenden Wirkstoff umfasst, wobei der Untersuchungsbereich die Leber oder ein Teil der Leber eines Untersuchungsobjekts ist, wobei das Volumenelement eine erste Gewebeart und eine zweite Gewebeart umfasst, wobei es sich bei der ersten Gewebeart um Hepatozyten handelt,
- wobei die Steuereinheit (2) konfiguriert ist, die Empfangseinheit (1) zu veranlassen, mehrere Datenbank-Signalverläufe (VDS₁, ..., VDSᵢ) aus einer Magnetresonanz-Fingerprint-Datenbank (DB) zu empfangen, wobei jedem Datenbank-Signalverlauf (VDS₁, ..., VDSⱼ) als Datenbankwerte eine T1-Relaxationszeit und eine T2-Relaxationszeit in beiden Gewebearten zugeordnet ist,
- wobei die Steuereinheit (2) konfiguriert ist, die Signalvergleichseinheit (3) zu veranlassen, den Magnetresonanz-Signalverlauf ((x,y,z)(1)) mit den Datenbank-Signalverläufen (VDS₁, ..., VDSⱼ) zu vergleichen, einen Datenbank-Signalverlauf (VDSₖ) mit einer definierten Übereinstimmung zu identifizieren und die zu dem identifizierten Datenbank-Signalverlauf (VDSₖ) zugeordneten Datenbankwerte der T1- und T2-Relaxationszeiten für beide Gewebearten zu ermitteln,
- wobei die Steuereinheit (2) konfiguriert ist, eine Konzentration des Kontrastmittels in der ersten Gewebeart und eine Konzentration des Kontrastmittels in der zweiten Gewebeart für das Volumenelement aus den ermittelten T1- und T2-Relaxationszeiten zu errechnen,
- wobei die Steuereinheit (2) konfiguriert ist, die Ausgabeeinheit (4) zu veranlassen, die ermittelten T1- und T2-Relaxationszeiten und/oder die errechneten Konzentrationen des Kontrastmittels in der ersten Gewebeart und in der zweiten Gewebeart für das Volumenelement abzuspeichern und/oder auszugeben.

8. Computerprogrammprodukt umfassend ein Computerprogramm, das in einen Arbeitsspeicher eines Computers geladen werden kann und dort den Computer dazu veranlasst, folgende Schritte ausführen:
• Empfangen eines Magnetresonanz-Signalverlaufs ((x,y,z)(i)) für ein Volumenelement eines Untersuchungsbereichs aus einem Magnetresonanz-Fingerprinting-Verfahren unter Verwendung eines Kontrastmittels, wobei das Kontrastmittel Gadoxetsäure oder ein Salz der Gadoxetsäure als kontrastverstärkenden Wirkstoff umfasst, wobei der Untersuchungsbereich die Leber oder ein Teil der Leber eines Untersuchungsobjekts ist, wobei das Volumenelement eine erste Gewebeart und eine zweite Gewebeart umfasst, wobei es sich bei der ersten Gewebeart um Hepatozyten handelt, wobei das Kontrastmittel in der ersten Gewebeart einen anderen Zustand aufweist, als in der zweiten Gewebeart, wobei es sich bei dem Zustand um die molare T1-Relaxivität und/oder T2-Relaxivität handelt,
• Empfangen von Datenbank-Signalverläufen (VDS₁, ..., VDSⱼ) aus einer Magnetresonanz-Fingerprint-Datenbank (DB), wobei jedem Datenbank-Signalverlauf (VDS₁, ..., VDSⱼ) als Datenbankwerte eine T1-Relaxationszeit und eine T2-Relaxationszeit in beiden Gewebearten zugeordnet ist,
• Vergleichen des Magnetresonanz-Signalverlaufs ((x,y,z)(1)) mit den Datenbank-Signalverläufen (VDS₁, ..., VDSⱼ),
• Identifizieren eines Datenbank-Signalverlaufs (VDSₖ) mit einer definierten Übereinstimmung mit dem Magnetresonanz-Signalverlauf ((x,y,z)(i)),
• Ermitteln der zugehörigen Datenbankwerte der T1- und T2-Relaxationszeiten in beiden Gewebearten in dem Volumenelement anhand des identifizierten Datenbank-Signalverlaufs (VDSₖ),
• Errechnung der Konzentration des Kontrastmittels in der ersten Gewebeart und der Konzentration des Kontrastmittels in der zweiten Gewebeart für das Volumenelement aus den ermittelten T1- und T2-Relaxationszeiten,
• Ausgeben der ermittelten T1- und T2-Relaxationszeiten und/oder der errechneten Konzentrationen des Kontrastmittels in der ersten Gewebeart und in der zweiten Gewebeart für das Volumenelement

## Claims

1. Method comprising the following steps:
• providing a magnetic resonance fingerprint database (DB),
- wherein the magnetic resonance fingerprint database (DB) comprises database waveforms (CDS₁, ..., CDSⱼ) for a specific contrast agent in at least two different tissue types, a first tissue type and a second tissue type, wherein the contrast agent comprises gadoxetic acid or a salt of gadoxetic acid as contrast-enhancing active substance, the first tissue type being hepatocytes,
- wherein the contrast agent has a different state in the first tissue type than in the second tissue type, the state being the molar T1 relaxivity and/or T2 relaxivity,
• acquiring a magnetic resonance waveform ((x,y,z)(i)) for a volume element of an examination region by means of a magnetic resonance fingerprinting method using the contrast agent, wherein the volume element includes the first tissue type and the second tissue type, wherein the examination region is the liver or part of the liver of an examination object,
• receiving database waveforms (CDS₁, ..., CDSⱼ) from the magnetic resonance fingerprint database (DB), wherein each database waveform (CDS₁, ..., CDSⱼ) is matched to a T1 relaxation time and a T2 relaxation time in both tissue types as database values,
• comparing the magnetic resonance waveform ((x,y,z)(i)) with database waveforms (CDS₁, ..., CDSⱼ),
• identifying a database waveform (CDSₖ) having a defined correspondence with the magnetic resonance waveform ((x,y,z)(i)),
• determining the associated database values for the T1 and T2 relaxation times in both tissue types in the volume element, based on the identified database waveform (CDSₖ),
• calculating the concentration of the contrast agent in the first tissue type and the concentration of the contrast agent in the second tissue type for the volume element from the determined T1 and T2 relaxation times,
• outputting the determined T1 and T2 relaxation times and/or the calculated concentrations of the contrast agent in the first tissue type and in the second tissue type for the volume element.

2. Method according to Claim 1, wherein a molar relaxivity in the first tissue type is at least 1.5 times greater, preferably at least 2 times greater, even more preferably at least 2.5 times greater, than in the second tissue type.

3. Method according to Claim 2, wherein a molar relaxivity at a magnetic field strength of the basic magnetic field of at least 1.5 tesla, preferably at least 2 tesla, even more preferably at least 2.5 tesla, even more preferably at least 3 tesla, most preferably at a magnetic field strength of 0.4 tesla to at least 3 tesla, is in the first tissue type at least 1.5 times greater, preferably at least 2 times greater, even more preferably at least 2.5 times greater, than in the second tissue type.

4. Method according to any of Claims 1 to 3, wherein the contrast agent has a higher molar T1 relaxivity in the first tissue type than in the second tissue type.

5. Method according to any of Claims 1 to 4, wherein the contrast agent comprises Gd-EOB-DTP disodium.

6. Method according to any of Claims 1 to 5, wherein the first tissue type is healthy liver tissue and the second tissue type is diseased liver tissue.

7. System comprising
• a receiving unit (1),
• a control unit (2),
• a signal comparison unit (3) and
• an output unit (4),
- wherein the control unit (2) is configured to cause the receiving unit (1) to receive a magnetic resonance waveform ((x,y,z) (i)) for at least one volume element of an examination region, the magnetic resonance waveform ((x,y,z)(i)) having been generated in a magnetic resonance fingerprinting method using a contrast agent, wherein the contrast agent comprises gadoxetic acid or a salt of gadoxetic acid as contrast-enhancing active substance, wherein the examination region is the liver or part of the liver of an examination object, wherein the volume element comprises a first tissue type and a second tissue type, the first tissue type being hepatocytes,
- wherein the control unit (2) is configured to cause the receiving unit (1) to receive a plurality of database waveforms (CDS₁, ..., CDSⱼ) from a magnetic resonance fingerprint database (DB), wherein each database waveform (CDS₁, ..., CDSⱼ) is matched to a T1 relaxation time and a T2 relaxation time in both tissue types as database values,
- wherein the control unit (2) is configured to cause the signal comparison unit (3) to compare the magnetic resonance waveform ((x,y,z) (i)) with the database waveforms (CDS₁, ..., CDSⱼ), to identify a database waveform (CDSₖ) having a defined correspondence, and to determine for both tissue types the database values for the T1 and T2 relaxation times matched to the identified database waveform (CDSₖ);
- wherein the control unit (2) is configured to calculate from the determined T1 and T2 relaxation times a concentration of the contrast agent in the first tissue type and a concentration of the contrast agent in the second tissue type for the volume element,
- wherein the control unit (2) is configured to cause the output unit (4) to store and/or output the determined T1 and T2 relaxation times and/or the calculated concentrations of the contrast agent in the first tissue type and in the second tissue type for the volume element.

8. Computer program product comprising a computer program that can be loaded into a memory of a computer, where it causes the computer to execute the following steps:
• receiving a magnetic resonance waveform ((x,y,z)(i)) for a volume element of an examination region from a magnetic resonance fingerprinting method using a contrast agent, wherein the contrast agent comprises gadoxetic acid or a salt of gadoxetic acid as contrast-enhancing active substance, wherein the examination region is the liver or part of the liver of an examination object, wherein the volume element comprises a first tissue type and a second tissue type, the first tissue type being hepatocytes, wherein the contrast agent has a different state in the first tissue type than in the second tissue type, the state being the molar T1 relaxivity and/or T2 relaxivity,
• receiving database waveforms (CDS₁, ..., CDSⱼ) from a magnetic resonance fingerprint database (DB), wherein each database waveform (CDS₁, ..., CDSⱼ) is matched to a T1 relaxation time and a T2 relaxation time in both tissue types as database values,
• comparing the magnetic resonance waveform ((x,y,z)(i)) with the database waveforms (CDS₁, ..., CDSⱼ),
• identifying a database waveform (CDSₖ) having a defined correspondence with the magnetic resonance waveform ((x,y,z)(i)),
• determining the associated database values for the T1 and T2 relaxation times in both tissue types in the volume element, based on the identified database waveform (CDSₖ),
• calculating the concentration of the contrast agent in the first tissue type and the concentration of the contrast agent in the second tissue type for the volume element from the determined T1 and T2 relaxation times,
• outputting the determined T1 and T2 relaxation times and/or the calculated concentrations of the contrast agent in the first tissue type and in the second tissue type for the volume element.

## Revendications

1. Procédé, comprenant les étapes suivantes consistant à :
• fournir une base de données d'empreintes à résonance magnétique (DB),
- dans lequel la base de données d'empreintes à résonance magnétique (DB) comprend des courbes de signal (VDS₁, ..., VDSⱼ) pour un agent de contraste spécifique dans au moins deux types de tissu différents, à savoir un premier type de tissu et un deuxième type de tissu, dans lequel l'agent de contraste comprend du gadoxetate ou un sel du gadoxetate comme substance renforçant le contraste, le premier type de tissu correspondant à des hépatocytes,
- dans lequel l'agent de contraste présente dans le premier type de tissu un état différent de celui dans le deuxième type de tissu, l'état étant la relaxivité molaire T1 et/ou T2,
• détecter une courbe de signal à résonance magnétique (x, y, z) (i)) pour un élément de volume d'un domaine d'examen au moyen d'un procédé de prise d'empreintes à résonance magnétique en utilisant l'agent de contraste, dans lequel l'élément de volume comprend le premier type de tissu et le deuxième type de tissu, le domaine d'examen étant le foie ou une partie du foie d'un objet à examiner,
• recevoir des courbes de signal de base de données (VDS₁, ..., VDSⱼ) de la base de données d'empreintes à résonance magnétique (DB), dans lequel un temps de relaxation T1 ou un temps de relaxation T2 dans les deux types de tissu est associé en tant que valeur de base de données à chaque courbe de signal de base de données (VDS₁, ..., VDSⱼ),
• comparer la courbe de signal à résonance magnétique ((x,y,z) (i)) avec des courbes de signal de base de données (VDS₁, ..., VDSⱼ),
• identifier une courbe de signal de base de données (VDSₖ) ayant une concordance définie avec la courbe de signal à résonance magnétique ((x,y,z)(i)),
• établir les valeurs de base de données associées des temps de relaxation T1 et T2 dans les deux types de tissu dans l'élément de volume à l'aide de la courbe de signal de base de données (VDSₖ) identifiée,
• calculer la concentration de l'agent de contraste dans le premier type de tissu et la concentration de l'agent de contraste dans le deuxième type de tissu pour l'élément de volume à partir des temps de relaxation T1 et T2 établis,
• sortir les temps de relaxation T1 et T2 établis et/ou les concentrations calculées de l'agent de contraste dans le premier type de tissu et dans le deuxième type de tissu pour l'élément de volume.

2. Procédé selon la revendication 1, dans lequel une relaxivité molaire dans le premier type de tissu est supérieure au moins du facteur 1,5, de préférence au moins du facteur 2, de plus grande préférence au moins du facteur 2,5 à celle dans le deuxième type de tissu.

3. Procédé selon la revendication 2, dans lequel une relaxivité molaire à une intensité de champ magnétique du champ magnétique de base d'au moins 1,5 Tesla, de préférence d'au moins 2 Tesla, de plus grande préférence d'au moins 2,5 Tesla, de plus grande préférence d'au moins 3 Tesla, de la plus grande préférence à une intensité de champ magnétique de 0,4 Tesla à au moins 3 Tesla dans le premier type de tissu est supérieure au moins du facteur 1,5, de préférence au moins du facteur 2, de plus grande préférence au moins du facteur 2,5 à celle dans le deuxième type de tissu.

4. Procédé selon l'une quelconque des revendications 1 à 3, dans lequel l'agent de contraste présente dans le premier type de tissu une relaxivité molaire T1 supérieure à celle dans le deuxième type de tissu.

5. Procédé selon l'une quelconque des revendications 1 à 4, dans lequel l'agent de contraste comprend du Gd-EOB-DTPA disodique.

6. Procédé selon l'une quelconque des revendications 1 à 5, dans lequel le premier type de tissu est du tissu de foie sain et le deuxième type de tissu est du tissu de fois pathologique.

7. Système, comprenant
• une unité de réception (1),
• une unité de commande (2),
• une unité de comparaison de signal (3), et
• une unité de sortie (4),
- dans lequel l'unité de commande (2) est configurée pour amener l'unité de réception (1) à recevoir pour au moins un élément de volume d'un domaine d'examen une courbe de signal à résonance magnétique ((x,y,z)(i)), dans lequel la courbe de signal à résonance magnétique ((x,y,z) (i)) a été générée dans un procédé de prise d'empreintes à résonance magnétique en utilisant un agent de contraste, dans lequel l'agent de contraste comprend du gadoxetate ou un sel du gadoxetate comme substance de renforcement de contraste, dans lequel le domaine d'examen est le foie ou une partie du foie d'un objet à examiner, l'élément de volume comprenant un premier type de tissu et un deuxième type de tissu, le premier type de tissu correspondant à des hépatocytes,
- dans lequel l'unité de commande (2) est configurée pour amener l'unité de réception (1) à recevoir plusieurs courbes de signal de base de données (VDS₁, ..., VDSⱼ) d'une base de données d'empreintes à résonance magnétique (DB), dans lequel un temps de relaxation T1 ou un temps de relaxation T2 dans les deux types de tissu est associé en tant que valeur de base de données à chaque courbe de signal de base de données (VDS₁, ..., VDSⱼ),
- dans lequel l'unité de commande (2) est configurée pour amener l'unité de comparaison de signal (3) à comparer la courbe de signal à résonance magnétique ((x,y,z) (i)) avec les courbes de signal de base de données (VDS₁, ..., VDSⱼ) , à identifier une courbe de signal de base de données (VDSₖ) ayant une concordance définie, et à établir les valeurs de base de données des temps de relaxation T1 et T2, associées à la courbe de signal de base de données identifiée (VDSₖ) pour les deux types de tissu,
- dans lequel l'unité de commande (2) est configurée pour calculer une concentration de l'agent de contraste dans le premier type de tissu et une concentration de l'agent de contraste dans le deuxième type de tissu pour l'élément de volume à partir des temps de relaxation T1 et T2 établis,
- dans lequel l'unité de commande (2) est configurée pour amener l'unité de sortie (4) à enregistrer et/ou à sortir les temps de relaxation T1 et T2 établis et/ou les concentrations calculées de l'agent de contraste dans le premier type de tissu et dans le deuxième type de tissu pour l'élément de volume.

8. Produit de programme informatique, comprenant un programme informatique qui peut être chargé dans une mémoire vive d'un ordinateur et qui y amène l'ordinateur à exécuter les étapes suivantes consistant à :
• recevoir une courbe de signal à résonance magnétique ((x, y, z) (i)) pour un élément de volume d'un domaine d'examen selon un procédé de prise d'empreintes à résonance magnétique en utilisant l'agent de contraste, dans lequel l'agent de contraste comprend du gadoxetate ou un sel du gadoxetate comme substance de renforcement de contraste, dans lequel le domaine d'examen est le foie ou une partie du foie d'un objet à examiner, dans lequel l'élément de volume comprend un premier type de tissu et un deuxième type de tissu, - dans lequel le premier type de tissu correspond à des hépatocytes, dans lequel l'agent de contraste présente dans le premier type de tissu un état différent de celui dans le deuxième type de tissu, l'état étant la relaxivité molaire T1 et/ou T2,
• recevoir des courbes de signal de base de données (VDS₁, ..., VDSⱼ) d'une base de données d'empreintes à résonance magnétique (DB), dans lequel un temps de relaxation T1 ou un temps de relaxation T2 dans les deux types de tissu est associé en tant que valeur de base de données à chaque courbe de signal de base de données (VDS₁, ..., VDSⱼ),
• comparer la courbe de signal à résonance magnétique ((x,y,z)(i)) aux courbes de signal de base de données (VDS₁, ..., VDSⱼ),
• identifier une courbe de signal de base de données (VDSₖ) ayant une concordance définie avec la courbe de signal à résonance magnétique ((x,y, z)(i)),
• établir les valeurs de base de données associées des temps de relaxation T1 et T2 dans les deux types de tissu dans l'élément de volume à l'aide de la courbe de signal de base de données (VDSₖ) identifiée,
• calculer la concentration de l'agent de contraste dans le premier type de tissu et la concentration de l'agent de contraste dans le deuxième type de tissu pour l'élément de volume à partir des temps de relaxation T1 et T2 établis,
• sortir les temps de relaxation T1 et T2 établis et/ou les concentrations calculées de l'agent de contraste dans le premier type de tissu et dans le deuxième type de tissu pour l'élément de volume.
